# EUROPÄISCHE PATENTSCHRIFT

(11) **EP 0 781 748 B1**
(45) Veröffentlichungstag und Bekanntmachung des Hinweises auf die Patenterteilung: **18.08.1999**
(21) Anmeldenummer: 96120152.2
(22) Anmeldetag: 16.12.1996
(51) Int. Cl.: C07C 29/124, C07C 33/22

(54) **Verfahren zur kontinuierlichen Herstellung von Benzylalkohol**
Method for the continuous preparation of benzyl alcohol
Procédé pour la préparation continue d'alcool benzylique

(30) Priorität: 27.12.1995 DE 19548876
(43) Veröffentlichungstag der Anmeldung: 02.07.1997
(73) Patentinhaber: BAYER AG, 51368 Leverkusen (DE)
(72) Erfinder: Buysch, Hans-Josef, Dr., 47809 Krefeld (DE); Jansen, Ursula, Dr., 47799 Krefeld (DE); Ooms, Pieter, Dr., 47800 Krefeld (DE); Hoffmann, Erhard-Günther, Dr., 40883 Ratingen (DE); Schenke, Berd-Ulrich, 46236 Bottrop (DE)

(56) Entgegenhaltungen:
- EP-A- 0 064 486
- EP-A- 0 768 291
- DE-A- 19 520 612

## Beschreibung

Vorliegende Erfindung betrifft ein Verfahren zur Herstellung von Benzylalkohol durch Hydrolyse von Benzylchlorid mit Wasser bei höheren Temperaturen, dadurch gekennzeichnet, daß man Wasser und Benzylchlorid kontinuierlich in einen Reaktor mit Dispersionseinrichtungen einspeist, das Reaktionsprodukt nach unvollständigem Umsatz in eine organische und wäßrige Phase trennt, die organische Phase kontinuierlich in eine erste Destillationskolonne einspeist und in Benzylchlorid, das in den Reaktor zurückfließt, und Roh-Benzylalkohol auftrennt, der aus einer zweiten Destillationseinheit als Rein-Benzylalkohol nebst Beiprodukte enthaltenden Sumpf hervorgeht, die wäßrige, Salzsäure enthaltende Phase in einer kontinuierlich arbeitenden Extraktionsapparatur mit einem Lösungsmittel behandelt, das die in Wasser gelösten organischen Stoffe, besonders Benzylalkohol, aufnimmt, den Extrakt in einer dritten Destillationseinheit vom Lösungsmittel befreit, das wieder in den Extraktionsapparat fließt, den Sumpf der dritten Destillationseinheit in die erste Destillationsapparatur einspeist und die Salzsäure enthaltende wäßrige Phase nach der Extraktion in einer Chlorwasserstoff-Absorptions-Anlage leitet, um sie an Chlorwasserstoff aufzukonzentrieren und einer weiteren Verwendung oder einer Salzsäureelektrolyse zwecks Chlorgewinnung zuzuführen.

Über die Hydrolyse von Benzylchlorid ist bereits sehr viel berichtet worden. Mehrere Publikationen beschäftigen sich mit diesem Problem zwecks Aufklärung des Reaktionsmechanismus und Erarbeitung analytischer Methoden. Dabei werden in der Regel homogene Mischungen aus Benzylchlorid, Wasser und einem wasserlöslichen Lösungsvermittler wie Alkohol, Aceton, Dioxan oder Essigsäure verwendet; siehe z.B. J. Chem. Soc. 1954, 1840 ff; Z. Naturf. 1946 (1) 580-4, J. Chem. Soc. 1957, 4747 ff; Tetrahedron 1957 (1) 129-144; Rec. 48, 227 ff (1929) und 49, 697 ff (1930). In verschiedenen Fällen wird die Hydrolyse auch in Gegenwart von Alkalihydroxiden, Alkalicarbonaten oder Erdalkalicarbonaten durchgeführt, wie z.B. in J.Am.Chem. Soc. 62, 2481 (1940), Rec. 53, 891 ff und 869 ff (1934), wobei letztere Veröffentlichungen zeigen, daß die Hydrolyse in Gegenwart von basischen Verbindungen beschleunigt wird. In allen diesen Arbeiten wird auf die Isolierung und Identifizierung der Hydrolyseprodukte kein Wert gelegt.

Es gibt jedoch auch Untersuchungen der Benzylchloridhydrolyse mit Wasser allein, die eine Beschreibung der Hydrolyseprodukte beinhalten. Nach Ann. 139, 307 f (1866) geht Benzylchlorid bei 190°C mit Wasser vor allem in Kohlenwasserstoffe und Dibenzylether über.

Unter milderen Bedingungen (Ann. 196, 353 (1879) bei 100 bis 110°C und vollständigem Umsatz erhielt man - allerdings in sehr starker Verdünnung - eine Ausbeute an Benzylalkohol von 76 % d.Th.; der Rest waren Hochsieder, die nicht charakterisiert wurden.

Die Erkenntnis, daß die Umsetzung in Gegenwart von Alkalien rascher und glatter verläuft, hat im technischen Bereich früh zur Bestrebung geführt, die während der Hydrolyse von Benzylchlorid entstehende Salzsäure zu binden. So wird im DRP 484 662 empfohlen, die Reaktion in Gegenwart von Calciumcarbonat durchzuführen, und in der US-PS 2 221 882 wird in Gegenwart zunächst einer Sodalösung und nachfolgend einer Natronlauge gearbeitet. Die Ausbeute liegt deutlich über 90 % d.Th.

Natriumcarbonat als Base hat sich offenbar bewährt und wurde in der Folgezeit beibehalten. Die weiteren Entwicklungen auf diesem Gebiet befassen sich mit der technischen Ausgestaltung dieser basischen Hydrolyse von Benzylchlorid als kontinuierliches Verfahren wie in DE-OS 2 101 810, EP-A 64 486 und DD-PS 161 067 beschrieben.

Nachteile der alkalischen Hydrolyse jedoch sind die zusätzliche Verwendung von Natriumcarbonat, die Bildung von Natriumchlorid und der Anfall von großen Mengen an wäßrigen Ablaugen, die entsorgt werden müssen.

Die Aufgabe der vorliegenden Erfindung bestand also darin, ein neues Verfahren zu entwickeln, das die Zugabe von Natriumcarbonat und damit Kochsalz- und Abwasserbelastung vermeidet.

Es wurde nun gefunden, daß man dies praktisch mit mindestens gleicher oder besserer Ausbeute an Benzylalkohol erreicht, wenn man bei Temperaturen zwischen 80°C und 180°C Benzylchlorid mit der 10- bis 70fachen Molmenge an Wasser unter intensiver Durchmischung reagieren läßt und unvollständig umsetzt.

Nach dem Stand der Kenntnisse war das Ergebnis keinesfalls zu erwarten: Die bisher bekannt gewordenen Ausbeuten waren mäßig und die Art der Nebenprodukte nicht bekannt. Die unter milden Bedingungen bei 100°C bis 110°C erhaltenen 76 % Benzylalkohol (Ann. 196, 353 (1879)) wurden bei einem Molverhältnis von H₂O : Benzylalkohol von 190:1, d.h. einem Gewichtsverhältnis von 27 : 1 gewonnen; dies bedeutet, daß die erhaltene Salzsäure einerseits zwar eine sehr niedrige Konzentration hatte und daher auch kaum die Nebenproduktbildung in Gang setzten konnte, andererseits aber auch keinen Gebrauchswert besaß.

Darüber hinaus war unter diesen Voraussetzungen eine technisch untragbar niedrige Raum-Zeit-Ausbeute zu erwarten.

Andererseits mußte bei einer Verminderung des Molverhältnisses unter 190 : 1 auf die anspruchsgemäßen Werte von 10 bis 70 : 1 mit einer deutlich vermehrten Nebenproduktbildung gerechnet werden, weil die Salzsäurekonzentration und damit ihre Aktivität um ein Mehrfaches steigt.

Demgegenüber ergibt das erfindungsgemäße Verfahren eine Salzsäure höherer Konzentration als Beiprodukt, das sich weiterverwenden läßt.

Geeignetes Ausgangsprodukt für das erfindungsgemäße Verfahren ist Benzylchlorid, wie es allgemein durch Seitenkettenchlorierung zugänglich ist.

Das erfindungsgemäße Verfahren zur Herstellung von Benzylalkohol durch Hydrolyse von Benzylchlorid mit Wasser bei erhöhter Temperatur ist dadurch gekennzeichnet, daß man kontinuierlich
a) Benzylchlorid und Wasser im Molverhältnis von 1 : (10 bis 70) in einen Reaktor mit Dispergiereinrichtungen einspeist, worin eine Temperatur von 80 bis 180°C herrscht und darin bis zu einem Umsatz an Benzylchlorid von 30 bis 90 % reagieren läßt,
b) das Reaktionsprodukt nach Verlassen des Reaktors in eine wäßrige und organische Phase separiert,
c) die organische Phase in einer ersten Destillationsapparatur in Benzylchlorid und Roh-Benzylalkohol trennt,
d) dieses Benzylchlorid in den Reaktor zurückgibt,
e) den Roh-Benzylalkohol in einer zweiten Destillationseinheit zu Reinst-Benzylalkohol raffiniert, wobei ein die Beiprodukte (besonders Dibenzylether) enthaltender Sumpf anfällt,
f) die verdünnte Salzsäure enthaltende wäßrige Phase aus dem Separator in einer Extraktionsapparatur mit einem Lösungsmittel behandelt, das die in der verdünnten Salzsäure noch gelösten organischen Bestandteile, besonders Benzylalkohol, aufnimmt,
g) diesen Extrakt in einer dritten Destillationsapparatur vom Lösungsmittel befreit, das in den Extraktor zurückfließt,
h) den Sumpf aus der dritten Destillation in die erste leitet, um daraus den Benzylalkohol zu gewinnen und
i) die verdünnte wäßrige Salzsäure aus der Extraktion nach Abziehen restlicher Organica in eine HCl-Absorptionsanlage leitet, um sie aufzukonzentrieren und als Reinst-Salzsäure einer weiteren Verwendung oder einer Salzsäureelektrolyse zwecks Chlorgewinnung für die Toluol-Seitenkettenchlorierung zuzuführen.

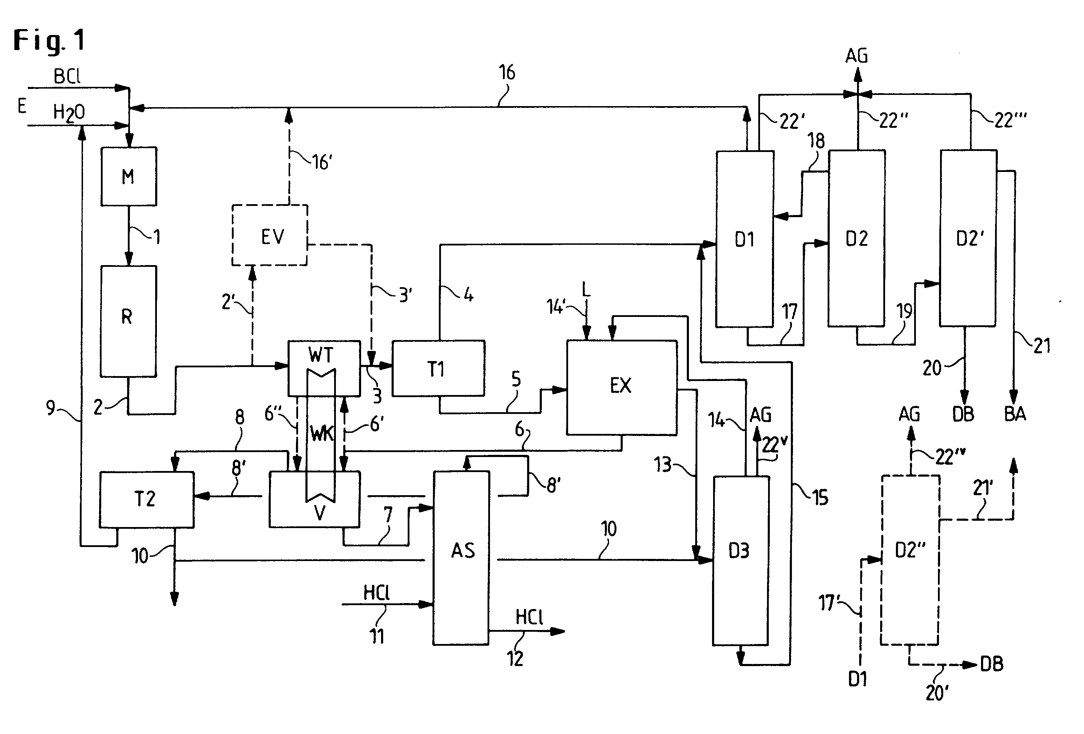

Das erfindungsgemäße Verfahren ist beispielhaft in Abb.1 dargestellt. Die Temperatur im Reaktor (R) beträgt 80 - 180°, bevorzugt 100 - 170°, besonders bevorzugt 110 - 150°C. Der Reaktor kann isotherm betrieben werden, indem man durch Kühlung die Reaktionswärme abführt oder aber vorteilhaft auch adiabatisch, indem man die Edukte E (BCl = Benzylchlorid und H₂O) mit einer solchen Temperatur über den Mischer (M) einführt (1), daß sich das Reaktionsgemisch im Reaktor auf die gewünschte Reaktionstemperatur aufheizt und mit dieser Temperatur auch den Reaktor verläßt (2).

Das Molverhältnis von Benzylchlorid (BCl) zu Wasser (H₂O) beträgt 1 : 10 bis 1 : 70; vorzugsweise 1 : 15 bis 1 : 55, besonders bevorzugt 1 : 20 bis 1 : 50, ganz besonders bevorzugt 1 : 25 bis 1 : 50.

Den Umsatz von Benzylchlorid bricht man zweckmäßig bei 35 bis 90 %, bevorzugt 40 bis 85 %, besonders bevorzugt 45 bis 80 % ab.

Als Reaktor (R) kann man einen einfachen Rührkessel verwenden, der das Reaktionsgemisch unter den Reaktionsbedingungen fein zu dispergieren vermag.

Zweckmäßiger verwendet man jedoch eine Rührkesselkaskade von 2 bis 7, vorzugsweise 3 bis 5 Kesseln.

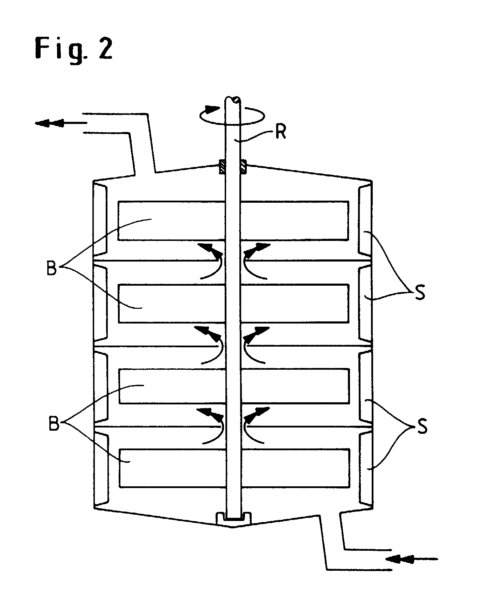

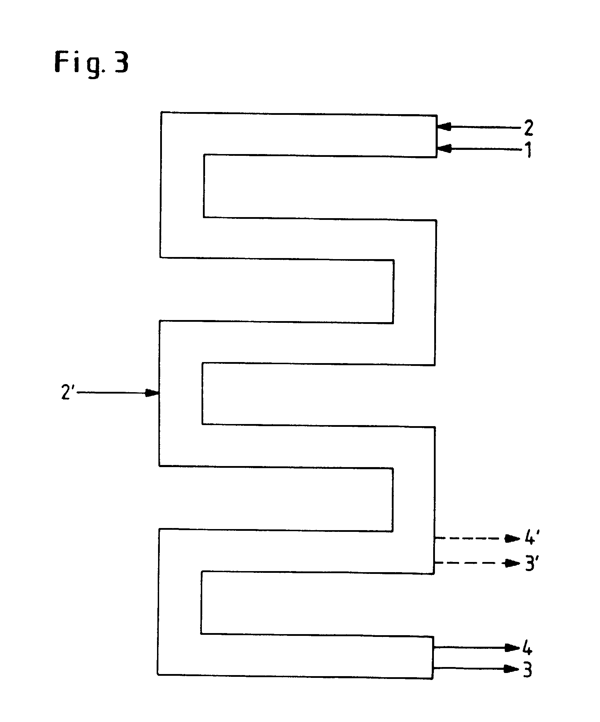

Vorteilhaft sind auch Kammerreaktoren (als Beispiel Abb. 2), in denen die einzelnen Kammern (K) gerührt werden mit einem Rührer (R), der 2-4 Blätter (B) je Kammer trägt, und die zur besseren Dispergierung mit 2 oder mehr Strombrechern (S) pro Kammer ausgestattet sind und unmittelbar miteinander verbunden sind, ohne daß nennenswerte Rückvermischung (beispielhafter Verlauf einer Strömung des zweiphasigen Gemisches) erfolgen kann (vgl. Abb. 2) mit 2 bis 10, bevorzugt 3 bis 8, besonders bevorzugt 3 bis 6 Kammern und Rohrreaktoren (Abb. 3), die das Reaktionsgemisch pfropfenförmig mit für die Entstehung eines turbulenten Zustandes hinreichender Geschwindigkeit durchströmt und in denen durch bekannte Misch- und Dispergierelemente Benzylchlorid in Wasser möglichst fein verteilt wird. Dabei kann das Reaktionsgemisch aus organischer (1) und wäßriger Phase (2) an beliebigen Stellen des Rohrreaktors ergänzt werden (2') und das Reaktionsgemisch aus Benzylchlorid und Benzylalkohol (3) und verdünnter Salzsäure (4) je nach Verweilzeit am Ende des Reaktors oder auch vorher (3' und 4') entnommen werden. Solche Rohrreaktoren können vom Fachmann nach bekannten Methoden berechnet und konstruiert werden (Perry's Chemical Engineers Handbook, Mc Graw Hill N.Y., 6th Ed. 1984).

Der Tröpfchendurchmesser der im Wasser dispergierten organischen Phase kann beispielsweise bei 100 - 400 µm, bevorzugt 150-300 µm liegen.

Wird die Reaktion bei Temperaturen >100°C durchgeführt, müssen druckfeste Apparate bis etwa 50 bar, vorzugsweise 30 bar, besonders bevorzugt 20 bar, verwendet werden.

Das den Reaktor verlassende Reaktionsgemisch (2) wird zweckmäßig bei erhöhter Temperatur zwischen 50 und 100°, vorzugsweise 55 bis 95° getrennt (T1) in eine organische Phase und in eine wäßrige verdünnte Salzsäure, deren Konzentration vom eingesetzten Molverhältnis der Reaktanten und dem erzielten Umsatz abhängt und beispielsweise 2 bis 7 Gew.-% HCl beträgt. Die Trennung der Phasen kann gegebenenfalls bedeutend durch Verwendung von Trennapparaten wie Koalescern beschleunigt werden, die beispielsweise in Ullmanns Encyclopedia of Ind. Chem. Unit Operations II Vol. B3 S. 6-31 und Chem. Ing. Techn. (1986) 58, 449 ff beschrieben sind.

Wenn die Reaktion bei Temperaturen >100°C, z.B. 130°C durchgeführt wird, kann das Reaktionsgemisch vor dem Eintritt in den Trennapparat (T1) durch Entspannungsverdampfung (2' → EV → 3') heruntergekühlt werden. Dabei kann ein Teil des nicht umgesetzten Benzylchlorids und ein Teil des Wassers aus dem Reaktionsgemisch verdampft und in den Reaktor zurückgeführt werden (16'). Auf diese Weise kann Energie für das Aufheizen der Edukte und Kühlflüssigkeit für das Reaktionsgemisch eingespart und die entstandene verdünnte Salzsäure etwas aufkonzentriert werden.

Es ist auch vorteilhaft, die Abkühlung des Reaktionsproduktes (2) vor dem Trennapparat (T1) dadurch zu bewerkstelligen, daß man in einem Wärmetauscher (WT) die überschüssige Wärme abführt und damit die den Extraktor (Ex) verlassende wäßrige Phase (6) entweder durch direkten Austausch (über 6' → 6'') in einem Wärmetauscher (WT) oder über einen Wärmeträgerkreislauf (WK) wieder aufheizt, um aus der wäßrigen Phase restliches organisches Material (8) zumindestens teilweise durch Abziehen (V) zu entfernen.

Die aus dem Trennapparat (T1) fließende organische Phase (4) enthält vor allem Benzylalkohol und Benzylchlorid, als Beiprodukt Dibenzylether und noch gelöst geringe Mengen verdünnter Salzsäure. Das Verhältnis der organischen Stoffe zueinander wird durch den im Reaktor erzielten Umsatz bestimmt. Der Gehalt an Benzylalkohol kann beispielsweise bei 40 - 75 %, an Dibenzylether bei 0,5 - 6 % und an Salzsäure bei 0,5 - 8 % liegen.

Die Auftrennung dieser Gemische durch Destillation (D1) ist nicht selbstverständlich, da bei höherer Temperatur aus Benzylchlorid und Benzylalkohol und aus Benzylalkohol und Salzsäure Nebenprodukte, besonders Dibenzylether, entstehen können. Diese Kondensationen erfolgen nach Literatur auch während der destillativen Aufarbeitung eines solchen Hydrolysegemisches, sobald der Gehalt an Benzylchlorid oberhalb von 1 % liegt (Chem. Prum. 32 (1982), 586; zitiert nach C.A. 98 106 890). Um diese während der Destillation auftretende nachträgliche Bildung von Dibenzylether zu unterdrücken, wird eine aufwendige Umsetzung von Resten an Benzylchlorid mit Stickstoffverbindungen, beispielsweise mit Hexamethylentetramin, empfohlen (CS 216 042 B; zitiert nach C.A. 102, 45606t). Die Trennung eines Gemisches, das Benzylchlorid, Benzylalkohol, Dibenzylether und wäßrige Salzsäure enthält, gelingt erfindungsgemäß dadurch, daß ein solches Gemisch einer kontinuierlich betriebenen Destillationskolonne mit Abtriebsteil und Verstärkerteil über eine Seiteneinspeisung zugeführt wird, die Destillationskolonne bei einem Druck von 1-950 mbar am Kopf der Kolonne betrieben wird und am Kopf der Destillationskolonne ein Gemisch, das im wesentlichen aus Benzylchlorid und wäßriger Salzsäure besteht, und aus dem Sumpf der Destillationskolonne ein Gemisch, das im wesentlichen aus Benzylalkohol und Dibenzylether besteht, entnommen werden.

Daher wird das den Trennapparat (T1) verlassende Gemisch (4) seitlich in eine erste Destillationskolonne (D1) eingespeist. Diese wird bei einem Druck von 1 - 950 mbar, bevorzugt 10 - 500 mbar, besonders bevorzugt 20 - 300 mbar am Kopf der Kolonne betrieben. Hierbei stellt sich in einer dem Fachmann bekannten Weise in Abhängigkeit vom eingestellten Kopfdruck und in Abhängigkeit von der Zusammensetzung des aufzutrennenden Gemisches eine Temperatur im Sumpf von 30 - 200°C, bevorzugt 60 - 180°C, besonders bevorzugt 70 - 165°C, ein. In entsprechender Weise stellt sich eine Kopftemperatur von 20 - 175°C, bevorzugt 50 - 155°C, besonders bevorzugt 60 - 140°C, ein. Die Kopftemperatur liegt hierbei stets unter der Sumpftemperatur. Die vorzunehmende Seiteneinspeisung des aufzutrennenden Gemisches wird an einem Punkt der Destillationskolonne vorgenommen, an welchem eine Temperatur von 25 - 195°C, bevorzugt 55 - 175°C, besonders bevorzugt 65 - 160°C, herrscht. Die Einstellung eines Temperaturgradienten ist dem Fachmann bekannt. Er ist u.a. abhängig von der Zusammensetzung des Gemisches sowie dessen Vorerwärmung. Die Destillationskolonne kann mit einer Belastung von 0,05 - 1,0 kg organische Phase im aufzutrennenden Gemisch pro Liter Leervolumen der Kolonne pro Stunde betrieben werden. Bevorzugt wird eine Belastung von 0,15 - 0,9 kg/l•h, besonders bevorzugt 0,25- 0,8 kg/l•h, eingestellt.

Die zusätzliche Bildung von Dibenzylether wird erfindungsgemäß praktisch verhindert und auf geringe Werte von weniger als 4 %, bevorzugt weniger als 2 %, besonders bevorzugt weniger als 0,5 %, bezogen auf die Menge an Benzylalkohol, gesenkt.

Am Kopf der Kolonne (D1) wird ein Gemisch (16) aus dem gesamten nicht umgesetzten Benzylchlorid und der gesamten noch gelösten Salzsäure, das in den Reaktor (R) zurückfließt und am Fuße der Kolonne ein Roh-Benzylalkohol (17), der praktisch kein Benzylchlorid mehr, aber alle Hochsieder, besonders Dibenzylether, enthält, abgenommen.

Die Kolonne kann mit den bekannten Einbauten wie Glockenböden, Siebböden und anderen Bödenkonstruktionen, mit Füllkörpern verschiedener Art oder besonders auch Packungen ausgerüstet sein.

Der Sumpf (17 bzw. 17') dieser Kolonne (D1) wird in eine zweite Destillationseinheit gefördert, die aus einer (D2") oder zwei (D2 + D2') Destillationskolonnen bestehen kann. Wird nur eine Kolonne (D2") verwendet, so muß das Sumpfprodukt (17) aus der Benzylchlorid-Destillation (D1) praktisch frei von Leichtsiedern insbesondere von Benzylchlorid sein. Am Kopf dieser Kolonne (D2") wird dann Reinst-Benzylalkohol (BA 21') mit einem Gehalt >99,99 % und aus dem Sumpf das Hochsiedergemisch (20') entnommen, das im wesentlichen aus Dibenzylether (DB), restlichem Benzylalkohol und wenig anderen hochsiedenden Material besteht und gegebenenfalls diskontinuierlich weiter aufgearbeitet werden kann.

Besteht die zweite Destillationseinheit aber aus zwei Kolonnen (D2 + D2') so kann in der ersten (D2) der Roh-Benzylalkohol (17) noch von geringen Mengen Leichtsiedern (18), besonders von Benzylchlorid, befreit werden, in der zweiten (D2') Kolonne wird dann der leichtsiederfreie Roh-Benzylalkohol (19) in Reinst-Benzylalkohol (21) und Sumpfprodukte (20), wie oben bereits beschrieben, aufgetrennt.

Nicht kondensierbare Leichtsieder und Abgase (22^{I}-22^{IV}) können aus den Destillationskolonnen entnommen und z.B. in einer Abgasverbrennung (AG) entsorgt werden.

Die Kolonnen D2-D2" werden im Vakuum bei 500 - 10 mbar, bevorzugt 250 - 10, besonders bevorzugt 100 - 10 mbar betrieben.

Die nach der Reaktion (2) im Trennapparat (T1) erhaltene verdünnte Salzsäure (5) wird in einer Extraktionseinrichtung (Ex) mit einem geeigneten Lösungsmittel behandelt, um die in der wäßrigen Phase gelösten organischen Bestandteile zu gewinnen - vor allem Benzylalkohol, der sich je nach Bedingungen zu ca. 2 - 4 % in der verdünnten Salzsäure (5) löst, und auch Benzylchlorid, das allerdings wenig gelöst ist. Die Temperatur bei der Extraktion (Ex) beträgt 20 - 100, vorzugsweise 40 - 95, besonders bevorzugt 50 - 95°C.

Geeignete Lösungsmittel sind Kohlenwasserstoffe mit 4 bis 9 Kohlenstoffatomen, Halogenkohlenwasserstoffe mit 1 bis 8 Kohlenstoffatomen, vorzugsweise aromatische Kohlenwasserstoffe wie Benzol, Toluol, Xylole, Ethylbenzol und Cumol, Halogenkohlenwasserstoffe wie Methylenchlorid, Chloroform, Dichlorethan, Trichlorethylen, Chlorbenzol und Benzylchlorid; besonders bevorzugt sind Toluol und Benzylchlorid.

Treten Verluste an Lösungsmittel (L) auf, können sie (14') in die Extraktion hinein ergänzt werden.

Die Extraktion (Ex) wird in bekannten Apparaten wie sie beispielsweise in Ullmann's Encyclopedia of Ind. Chem. Unit. Oper. II Vol. B3, S. 6-14 ff beschrieben sind, im Gegenstrom kontinuierlich durchgeführt. Genannt seien Füllkörperkolonnen, Bodenkolonnen, pulsierende Bodenkolonnen, pulsierende Packungskolonnen, Mixer-Settler-Batterien, Kolonnen mit rotierenden Einbauten und Zentrifugal-Extraktoren.

Der Extrakt (13) wird vom Extraktionsmittel und Spuren der verdünnten Salzsäure in einer dritten Destillationskolonne (D3) befreit, das Kopfprodukt (14) wieder in den Extraktionsapparat (Ex) gegeben und der Sumpf (15) aus dieser Kolonne (D3) zusammen mit (4) eingespeist, in die erste Destillationskolonne (D1), wo Benzylalkohol und Benzylchlorid getrennt werden. Diese dritte Destillationskolonne (D3) arbeitet bei 750 - 50, bevorzugt 500 - 150 und besonders bevorzugt 400 - 200 mbar.

Mit der verdünnten wäßrigen Salzsäure (6) aus dem Extraktor (Ex) wird in bekannter Weise eine HCl-Absorptionsanlage (AS) betrieben, in der beispielsweise Chlorwasserstoff (11) aus einer Toluol- oder Aromatenchlorierung in eine hochprozentige Salzsäure (12) übergeführt wird, die sehr rein und für anderweitige technische Anwendungen oder eine elektrolytische Aufarbeitung zu Chlor geeignet ist.

Bevor die verdünnte Salzsäure (6) aus der Extraktion (Ex) in die Absorptionsanlage (AS) geleitet wird, sollten zweckmäßig darin vorhandene Reste organischer Verbindungen, die von der Extraktion (Ex) her noch in der Salzsäure (6) gelöst sind, wie z.B. Toluol, Benzylchlorid oder Spuren Benzylalkohol, möglichst weitgehend durch Abziehen (V) abgetrennt werden. Die von Organica praktisch freie verdünnte Salzsäure (7) wird dann in der Absorption (AS) durch Destillation vollständig von Spuren Kohlenstoffverbindungen befreit und das Destillat (8') in einer Trennanlage (T2) in Wasser und Organica separiert.

Wie schon beschrieben, wird der Stripper (V) mit der Abwärme aus dem Reaktionsgemisch (2) über Wärmeaustausch (WT direkt oder über WK) versorgt. Den Stripper verläßt ein Gemisch (8) aus überwiegend Wasser und wenig organischem Material, das im Trenngefäß (T2) zusammen mit dem gegebenenenfalls aus der Absorption (AS) kommenden Gemisch (8') aus Wasser und Resten Organica in eine wäßrige und organische Phase getrennt wird. Die wäßrige Phase (9) fließt über (M) zurück in die Reaktion (R), die organische Phase (10) kann in eine Toluolchlorierung oder wieder in die dritte Destillationskolonne (D3) eingespeist werden.

Auf diese Weise wird ein kontinuierliches Verfahren zur Herstellung von Benzylalkohol aus Benzylchlorid durch Hydrolyse mit Wasser durchgeführt, das keine Basen benötigt, weder Salze noch Ablaugen erzeugt, die entsorgt werden müssen, noch nennenswerte Mengen an organisch-chemischen Abfallprodukten, denn Dibenzylether kann entweder direkt industriell z.B. in der Kautschukindustrie verwendet oder aber zusammen mit den anderen Hochsiedern (20 + 20') und den organischen Verbindungen (10) aus dem Trenngefäß (T2) in einer Toluolchlorierung weiter mit Chlor zu Benzotrichlorid und Benzoylchlorid umgesetzt werden.

Benzylalkohol ist ein wichtiges, wegen seiner äußerst geringen Toxizität an Bedeutung zunehmendes Produkt für die Herstellung von Parfüms, Kosmetika, Lacken, Weichmachern und ein begehrtes Hilfsmittel für die Kautschuk-, Farben- und Textilindustrie.

Die Prozentangaben in den nachfolgenden Beispielen beziehen sich jeweils auf das Gewicht; Teile sind Gewichtsteile.

### Beispiel

Benzylchlorid und Wasser wurden im Molverhältnis 1 35 bei 130°C miteinander reagieren gelassen, die Reaktion wurde bei einem Benzylchloridumsatz von 60 % abgebrochen und das Reaktionsgemisch aufgearbeitet.

Die Durchführung dieses Beispiels wird anhand der Abb. 1 erläutert. Die folgenden Mengen wurden jeweils pro Stunde durch die Anlage durchgesetzt:
- 14,01 Tle.: Benzylchlorid (BCl) von ca. 35°C, E,
- 96,75 Tle.: Wasser von ca. 150°C, E, dazu
- 10,51 Tle.: eines Gemisches 16 von ca. 40°C, das Kopfprodukt der Destillationskolonne D1, bestehend aus ca. 88 % Benzylchlorid, 4 % Benzylalkohol, 6 % Wasser und Spuren HCl und Toluol und etwa
- 18,9 Tle.: eines Gemisches 9 von ca. 40°C, das zu >99 % aus Wasser und nur in Spuren aus Benzylchlorid und Benzylalkohol bestand und aus dem Trennbehälter T2 stammte,
wurden unter einem Druck von ca. 10 bar durch einen Mischer M gepumpt und dann durch eine Kaskade aus 5 hintereinandergeschalteten, mit Strombrechern ausgerüsteten und intensiv gerührten Rührbehältern, wobei die Temperatur bei guter Isolierung auf 130°C stieg.

Nach Verlassen der Kaskade enthielt das Reaktionsgemisch ca. 81 % Wasser, 6,6 % Benzylchlorid, 8,5 % Benzylalkohol, 0,27 % Hochsieder (vor allem Dibenzylether) und etwa 2,9 % HCl.

Die Mischung gelangte über 2 in den Wärmetauscher WT, wo sie auf ca. 90°C abgekühlt wurde, in das Trenngefäß mit Koalescer T1, in dem sich die organische Phase von der wäßrigen Phase absetzte. Letztere (etwa 121 Tle., ca. 94 % H₂O, 3,3 % HCl, 2,6 % Benzylalkohol und Spuren Benzylchlorid, Toluol und Dibenzylether) wurde im Extraktor Ex, einer Pulsationskolonne, mit Toluol extrahiert. Die ablaufende wäßrige Phase 6 mit ca. 96 % Wasser, 0,1 % Benzylalkohol, 3,4 % HCl, etwas Toluol und Spuren Benzylchlorid wurde im Verdampfer V mit der Abwärme des Reaktionsgemisches über den Wärmetauscher WT gestrippt und weiter von den restlichen Organica befreit, so daß sie durch 7 in den HCl-Absorber AS abfließen konnte, aus dem die letzten Spuren organischen Materials durch Destillation über 8' entnommen wurden. Diese Destillate 8' und 8 aus V und AS wurden im Trennbehälter T2 von den abgestrippten organischen Verbindungen 10 (0,15 - 0,2 Tle.) getrennt, die in die Kolonne D3 oder nach Ex geleitet wurden. Die wäßrige Phase (>99 %) 9 aus T2 floß in den Mischer M zurück.

Verluste an Toluol L (0,2 - 0,4 Tle.) wurden aus einem Vorratsgefäß über 14' in Ex ergänzt.

Der toluolische Extrakt aus Ex gelangte durch 13 in die Eindampfkolonne D3, aus der bei 200 - 250 mbar Toluol über Kopf und dann (14) wieder in die Extraktion ging. Das Sumpfprodukt mit ca. 94 % Benzylalkohol, 3,6 % Hochsiedern und Resten Toluol und Benzylchlorid wurde zusammen mit der organischen Phase 4 aus T1 in der Kolonne D1 fraktioniert.

Diese aus dem Trennapparat T1 außer der wäßrigen Phase 5 laufende organische Phase 4 enthielt die Hauptmenge des organischen Reaktionsproduktes, nämlich etwa 19 Tle. mit ca. 48 % Benzylchlorid, 46 % Benzylalkohol, 1,5 % Hochsieder, besonders Dibenzylether, 3,5 % Wasser, <1 % Toluol und um 0,1 % HCl. In der Destillationskolonne D1 wurden sie zusammen mit dem Sumpfprodukt 15 aus der Kolonne D3 (ca. 3 Tle.) destillativ aufgearbeitet. Bei 100 - 150 mbar gingen über Kopf 10,5 Tle., die zu >88 % aus Benzylchlorid, ca. 4 % Benzylalkohol, 6 % Wasser, 1 % Toluol und dem eingetragenen HCl bestanden und wieder über 16 dem Mischer M und damit der Reaktion zuflossen.

Aus dem Sumpf der Kolonne D1 liefen etwa 12,5 Tle. roher Benzylalkohol mit einem Gehalt von ca. 97 %, 3 % Hochsieder und ca. 10 ppm Benzylchlorid ab. Durch die Zuleitung 17 wurde dieses Sumpfprodukt in die Kolonne D2 eingespeist, wo bei 30 - 40 mbar eine Abtrennung der letzten Leichtsieder stattfand, die über 18 in die Kolonne D1 zurückflossen. Das Sumpfprodukt aus D2 (ca. 11,8 Tle.) wurde dann durch 19 in die Kolonne D2' gepumpt und endgültig bei 30 - 50 mbar zu Reinst-Alkohol (11,2 - 11,3 Tle.; >99,999 %ig) aufgearbeitet. Der verbleibende Sumpf (0,5 - 0,6 Tle.) enthielt noch etwa 25 % Benzylalkohol, der in einer diskontinuierlich arbeitenden Vakuumdestillation isoliert und in die Kolonne D2 oder D2' zurückgeführt werden konnte. Damit stieg die Ausbeute auf 11,4 - 11,5 Tle.

Der Hochsiedersumpf 20 konnte in einer Chlorierungsapparatur in Benzotrichlorid und Benzoylchlorid überführt werden. Über die Abgasleitungen 22' bis 22^{v} entstand ein geringer Verlust, der besonders aus Toluol und Wasser bestand und in eine Verbrennungsanlage geleitet wurde.

Aus 14,01 Tlen/h Benzylchlorid enstanden somit ca. 11,2 bzw. nach Rückgewinnung des Benzylalkohols aus dem Sumpf von D2' bis zu 11,5 Tle. Benzylakohol/h.

Dies entspricht einer Ausbeute von 94 bis 96 % d.Th.

Nicht verwertbare Neben- und Beiprodukte wurden praktisch nicht erhalten.

## Patentansprüche

1. Verfahren zur Herstellung von Benzylalkohol durch Hydrolyse von Benzylchlorid mit Wasser bei erhöhter Temperatur dadurch gekennzeichnet, daß man kontinuierlich
a) Benzylchlorid und Wasser im Molverhältnis von 1 : (10 bis 70) in einen Reaktor mit Dispergiereinrichtungen einspeist, worin eine Temperatur von 80 bis 180°C herrscht und darin bis zu einem Umsatz an Benzylchlorid von 30 bis 90 % reagieren läßt,
b) das Reaktionsprodukt nach Verlassen des Reaktors in eine wäßrige und organische Phase separiert,
c) die organische Phase in einer kontinuierlich betriebenen Destillationskolonne mit Abtriebsteil und Verstärkerteil über eine Seiteneinspeisung zu - führt , die Destillationskolonne bei einem Druck von 1-950 mbar am Kopf der Kolonne betrieben wird und am Kopf der Destillationskolonne ein Gemisch, das im wesentlichen aus Benzylchlorid und wäßriger Salzsäure besteht, und aus dem Sumpf der Destillationskolonne ein Gemisch, das im wesentlichen aus Benzylalkohol und Dibenzylether besteht, enommen werden,
d) dieses Benzylchlorid in den Reaktor zurückgibt,
e) den Roh-Benzylalkohol in einer zweiten Destillationseinheit zu Reinst-Benzylalkohol raffiniert, wobei ein die Beiprodukte (besonders Dibenzylether) enthaltender Sumpf anfällt,
f) die verdünnte Salzsäure enthaltende wäßrige Phase aus dem Separator in einer Extraktionsapparatur mit einem Lösungsmittel behandelt, das die in der verdünnten Salzsäure noch gelösten organischen Bestandteile, besonders Benzylalkohol, aufnimmt,
g) diesen Extrakt in einer dritten Destillationsapparatur vom Lösungsmittel befreit, das in den Extraktor zurückfließt,
h) den Sumpf aus der dritten Destillation in die erste leitet, um daraus den Benzylalkohol zu gewinnen und
i) die verdünnte wäßrige Salzsäure aus der Extraktion nach Abziehen restlicher Organica in eine HCl-Absorptionsanlage leitet, um sie aufzukonzentrieren und als Reinst-Salzsäure einer weiteren Verwendung oder einer Salzsäureelektrolyse zwecks Chlorgewinnung für die Toluol-Seitenkettenchlorierung zuzuführen.

## Claims

1. Process for the preparation of benzyl alcohol by hydrolysis of benzyl chloride with water at elevated temperature characterized in that
a) benzyl chloride and water are continuously fed into a reactor containing dispersion devices in a molar ratio of 1 : (10 to 70), in which reactor a temperature of 80 to 180°C prevails, and are reacted therein up to a benzyl chloride conversion rate of 30 to 90%,
b) the reaction product, after exiting the reactor, is separated into an aqueous and organic phase,
c) the organic phase is fed via a side feed to a continuously operating distillation column having a stripping section and an enrichment section, the distillation column is operated at a pressure of 1-950 mbar at the top of the column and a mixture which essentially comprises benzyl chloride and aqueous hydrochloric acid is taken off at the top of the distillation column and a mixture which essentially comprises benzyl alcohol and dibenzyl ether is taken off from the bottom of the distillation column,
d) this benzyl chloride is recycled to the reactor,
e) the crude benzyl alcohol is refined in a second distillation unit to give high-purity benzyl alcohol, a bottom phase containing the by-products (particularly dibenzyl ether) being produced,
f) the aqueous phase from the separator containing dilute hydrochloric acid is treated in an extraction apparatus with a solvent which takes up the organic constituents, particularly benzyl alcohol, which are still dissolved in the dilute hydrochloric acid,
g) in a third distillation apparatus, this extract is freed of solvent which returns to the extractor,
h) the bottom phase from the third distillation is passed to the first, in order to recover the benzyl alcohol therefrom and
i) the dilute aqueous hydrochloric acid from the extraction, after taking off residual organics, is passed into an HCl absorption unit in order to concentrate it and feed it as high-purity hydrochloric acid to further use or to hydrochloric acid electrolysis for chlorine production for toluene side-chain chlorination.

## Revendications

1. Procédé de préparation d'alcool benzylique par hydrolyse de chlorure de benzyle avec de l'eau à température élevée, caractérisé en ce que, de manière continue l'on :
a) alimente en chlorure de benzyle et en eau, en un rapport molaire de 1:(10 à 70), un réacteur avec dispositifs de dispersion, où il règne une température allant de 80 à 180°C et dans lequel on laisse réagir jusqu'à une transformation du chlorure de benzyle allant de 30 à 90% ;
b) sépare le produit de réaction après avoir quitté le réacteur, en une phase aqueuse et une phase organique ;
c) conduit la phase organique dans une colonne de distillation fonctionnant en continu avec une partie de rectification et une partie d'amplification par introduction latérale, la colonne de distillation fonctionne à une pression allant de 1 à 950 mbar à la tête de la colonne et on prélève à la tête de la colonne, un mélange qui consiste essentiellement en chlorure de benzyle et acide chlorhydrique aqueux, et à la queue de la colonne de distillation, un mélange qui consiste essentiellement en alcool benzylique et dibenzyléther ;
d) renvoie ce chlorure de benzyle dans le réacteur ;
e) raffine l'alcool benzylique brut dans une deuxième unité de distillation en alcool benzylique pur, où une queue contenant les produits secondaires (particulièrement le dibenzyléther) se forme;
f) traite la phase aqueuse contenant l'acide chlorhydrique dilué du séparateur dans un appareil d'extraction avec un solvant qui recueille les constituants organiques encore dissous dans l'acide chlorhydrique dilué, en particulier l'alcool benzylique ;
g) libère cet extrait, dans un troisième appareil de distillation, du solvant qui est reflué dans l'extracteur ;
h) conduit la queue de la troisième distillation dans la première pour en obtenir l'alcool benzylique, et
i) conduit l'acide chlorhydrique aqueux dilué de l'extraction après élimination des composés organiques restants, dans une installation d'absorption d'HCl, pour le concentrer et l'envoyer sous forme d'acide chlorhydrique pur, vers une autre utilisation ou une électrolyse d'acide chlorhydrique pour produire du chlore pour la chloration de chaîne latérale du toluène.
